# EUROPÄISCHE PATENTSCHRIFT

(11) **EP 0 531 355 B1**
(45) Veröffentlichungstag und Bekanntmachung des Hinweises auf die Patenterteilung: **13.09.1995**
(21) Anmeldenummer: 91909738.6
(22) Anmeldetag: 21.05.1991
(51) Int. Cl.: C07C 309/17, C07C 303/32

(54) **VERFAHREN ZUR HERSTELLUNG HOCHKONZENTRIERTER PASTEN VON $g(a)-SULFOFETTSÄUREALKYLESTER-ALKALIMETALLSALZEN**
PROCESS FOR PRODUCING HIGHLY CONCENTRATED PASTES OF $g(a)-SULPHO FATTY ACID ALKYL ESTER ALKALI METAL SALTS
PROCEDE DE PRODUCTION DE PATES TRES CONCENTREES DE SELS DE METAUX ALCALINS-ALKYLESTERS D'ACIDES GRAS $g(a)-SULFONIQUES

(30) Priorität: 30.05.1990 DE 4017463
(43) Veröffentlichungstag der Anmeldung: 17.03.1993
(73) Patentinhaber: Henkel Kommanditgesellschaft auf Aktien, 40191 Düsseldorf (DE)
(72) Erfinder: COLIGNON, Dietmar, D-4006 Erkrath (DE); DORRA, Erich, D-4000 Düsseldorf 13 (DE); PANTEL, Günter, D-5657 Haan (DE); SCHMIDT, Wolfgang, D-4019 Monheim 2 (DE); WREDE, Norbert, D-4000 Düsselforf 13 (DE)
(86) Internationale Anmeldenummer: EP9100942
(87) Internationale Veröffentlichungsnummer: WO9118873

(56) Entgegenhaltungen:
- EP-A- 0 182 118

## Beschreibung

Die Erfindung betrifft ein Verfahren zur Herstellung frei beweglicher Pasten von α-Sulfofettsäurealkylester-Alkalimetallsalzen mit Waschaktivsubstanzgehalten von 60 bis 70 Gew.-%, bei dem man sauren α-Sulfofettsäurealkylester unter Einhaltung von definierten pH-Bedingungen mit wässrigen Alkalimetallhydroxidlösungen neutralisiert. Unter Waschaktivsubstanz (WAS) wird im Zusammenhang mit der Erfindung die Summe aus α-Sulfofettsäurealkylester-Alkalimetallsalz und dem stets als Nebenprodukt vorhandenen α-Sulfofettsäure-Dialkalimetallsalz im neutralisierten α-Sulfofettsäureester verstanden.

α-Sulfofettsäurealkylester-Alkalimetallsalze haben derzeit steigende Bedeutung als oberflächenaktive Substanzen für Wasch- und Reinigungsmittel aus nachwachsenden natürlichen Rohstoffen. Man erhält die α-Sulfofettsäurealkylester-Alkalimetallsalze nach bekannten Verfahren in Form von wässrigen Lösungen oder Pasten durch Neutralisation von α-Sulfofettsäurealkylestern, welch letztere durch Umsetzung von Fettsäureniedrigalkylestern mit gasförmigem SO₃ synthetisiert werden können. Als Basis für die Herstellung der α-Sulfofettsäurealkylester-Alkalimetallsalze dienen letzten Endes Fette und Öle natürlichen Ursprungs, aus denen die Fettsäureniedrigalkylester durch Fettspaltung und nachfolgende Veresterung der freien Fettsäuren mit niederen Alkanolen oder durch Umesterung der natürlichen Triglyceride mit niederen Alkanolen erhalten werden. Bei beiden Reaktionen ist Methanol als niederes Alkanol bevorzugt. Die Fettsäureniedrigalkylester stellen Gemische dar, in denen Fettsäurereste mit 6 bis 22 Kohlenstoffatomen vorkommen, wobei die Kettenlängenverteilung vom Ursprung der natürlichen Fette oder Öle abhängig ist. Diese Fettsäureestergemische werden häufig nicht als solche, sondern in Form bestimmter Fraktionen zur Synthese eingesetzt. Durch Sulfonierung der Fettsäureestergemische mit gasförmigem SO₃ erhält man saure α-Sulfofettsäurealkylester, die durch Neutralisation auf einen pH-Wert von 6 bis 8 in wässrige Pasten von α-Sulfofettsäurealkyleseter-Alkalimetallsalzen überführt werden. Die rohen α-Sulfofettsäurealkylester und gegebenenfalls auch ihre Alkalimetallsalze stellen mehr oder weniger gefärbte Produkte dar, die in der Regel vor und/oder nach der Neutralisation einer Behandlung mit üblichen Bleichmitteln wie Wasserstoffperoxid oder Alkalimetallhypochlorit unterworfen werden müssen.

Eine besondere Schwierigkeit bei der Herstellung und Handhabung von wässrigen α-Sulfofettsäurealkylester-Alkalimetallsalzpasten ergibt sich aus ihrem Viskositätsverhalten in Abhängigkeit von der Feststoffkonzentration. Die nach den gängigen technischen Verfahren hergestellten α-Sulfofettsäurealkylester-Alkalimetallsalze, im Folgenden auch kurz als Estersulfonate bezeichnet, bilden in wässrigen Zusammensetzungen nur bei WAS-Gehalten bis zu etwa 40 Gew.-% und dann wieder ab WAS-Gehalten von etwa 55 Gew.-% Lösungen oder Suspensionen mit so geringer Viskosität, daß eine hinreichende Beweglichkeit gegeben ist, um einen störungsfreien Ablauf technischer Vorgänge zu gewährleisten. In dem dazwischen liegenden Konzentrationsbereich, also bei WAS-Gehalten von etwa 40 bis 55 Gew.-% zeigen die wässrigen Estersulfonat-Zusammensetzungen extrem hohe Viskositätswerte und stellen sich als mehr oder weniger feste Gele dar, die nicht gerührt oder umgepumpt werden können. Die Unter- und Obergrenzen der individuellen Viskositätsmaxima können im übrigen jeweils um ± 5 Gew.-%. WASGehalt schwanken. Dieses besondere Konzentrations-/Viskositätsverhalten bedingt, daß durch einfache Neutralisation der sauren α-Sulfofettsäurealkylester mit der berechneten Menge wässriger Alkalimetallhydroxidlösungen keine Estersulfonatpasten mit WAS-Gehalten über 35 bis 40 Gew.-% eingestellt werden können. Nach dem Überschreiten der Untergrenze des Viskositätsmaximums geht die Rührbarkeit und Vermischbarkeit des reagierenden Gemisches verloren. Die mangelnde Rühr- und Mischbarkeit verhindert einen hinreichenden schnellen Abtransport der Neutralisationswärme. Durch lokale Konzentrations- und Temperaturspitzen werden unerwünschte Nebenreaktionen ausgelöst, insbesondere die Spaltung der in den Estersulfonaten vorhandenen Esterbindungen, wodurch im Endprodukt unerwünscht hohe Konzentrationen an Alkalimetall-Disalzen der freien α-Sulfofettsäuren entstehen. Selbstverständlich ist auch die weitere Verarbeitung von Estersulfonatpasten, die durch den hohen Viskositätsanstieg immobilisiert sind, bis hin zur Undurchführbarkeit beeinträchtigt, allein schon durch die Tatsache, daß derartige wässrige Zusammmensetzungen nicht mehr gieß- und pumpfähig sind.

Das Entstehen von Disalzen der freien α-Sulfofettsäurealkylester ist aus mehreren Gründen unerwünscht. Die Disalze sind in Wasser nur im beschränkten Umfang löslich und zeigen darüber hinaus unzureichende oberflächenaktive Eigenschaften. Vor allem aber haben Disalze als Nebenprodukte in Estersulfonatpasten eine beträchtliche viskositätssteigernde Wirkung.

Es hat in der Vergangenheit nicht an Versuchen gefehlt, die durch das besondere Konzentrations-/Viskositätsverhalten der Estersulfonate und das unerwünschte Entstehen von α-Sulfofettsäure-Disalzen bedingten nachteiligen Erscheinugen wenigstens weitgehend ausschalten. So wurde vorgeschlagen, das Fließverhalten von wässrigen Estersulfonat-Zusammensetzungen durch den Zusatz von Fließhilfsmitteln zu verbessern. Nach der DE-OS 33 05 430 werden als Viskositätsregler aliphatische Alkohole mit 8 bis 40 Kohlenstoffatomen und 1 bis 6 Hydroxylgruppen, Alkylphenole und Anlagerungsprodukte von bis zu 20 Mol Ethylenoxid und/oder Propylenoxid an die genannten Alkohole und Alkylphenole eingesetzt.

Im Zusammenhang mit der unerwünschten Bildung von Disalzen bei der Aufarbeitung der saueren α-Sulfofettsäurealkylester beschreibt die DE-OS 31 23 681 ein Verfahren, bei dem die Neutralisation in zwei Stufen durchgeführt wird. In der ersten Neutralisationsstufe wird dort mit einer 15 bis 50 gew.-%igen Alkalimetallhydroxidlösung in Gegenwart eines Alkohols mit 1 bis 4 Kohlenstoffatomen, vorzugsweise Methanol, in einer Menge von 5 bis 20 Gew.-%, bezogen auf das Gewicht des sulfonierten Produktes, bis auf einen pH-Wert von 2,5 bis 4 neutralisiert, bevor in der zweiten Neutralisationsstufe mit Hilfe einer stärker verdünnten Alkalimetallhydroxidlösung ein End-pH-Wert von 6 bis 7 eingestellt wird. Mit Hilfe dieses Verfahrens soll es möglich sein, in den Estersulfonat-Zusammensetzungen den Disalzgehalt auf 5 Gew.-%, bezogen auf Waschaktivsubstanz, oder weniger zurückzudrängen. Ein gravierender Nachteil dieses Verfahrens liegt auf der Hand: Die auf diese Weise hergestellten Estersulfonatpasten enthalten beträchtliche Mengen Alkohol, die bei der Herstellung von Waschmittelgemischen durch Sprühtrocknung insofern stören, als sie das unerwünschte "Pluming" auslösen können. Zur Begrenzung des Alkoholgehaltes in den Endprodukten schlägt die DE-OS 33 34 517 vor, die gegebenenfalls durchgeführte Bleiche und die Neutralisation der rohen α-Sulfofettsäurealkylester in Gegenwart einer solchen Menge eines niederen Alkohols vorzunehmen, daß eine wässrige Aufschlämmung mit 30 bis 40 Gew.-% und - bezogen auf das Gewicht des α-Sulfofettsäureestersalzes - 5 bis 15 Gew.-% eines niederen Alkoholsulfates sowie 8 bis 40 Gew.-% des niederen Alkohols erhalten wird. Abschließend soll die wässrige Aufschlämmung derart eingeengt werden, daß sie 40 bis 65 Gew.-% α-Sulfofettsäureestersalz, 2 bis 10 Gew.-% niederes Alkoholsulfat und höchstens 2 Gew.-% niederen Alkohol enthält.

Nach der DE-OS 34 32 324 läßt sich der Disalzgehalt in α-Sulfofettsäurealkylester-Alkalimetallsalz-Pasten dadurch regeln und senken, daß man das rohe Sulfonierungsprodukt vor der Behandlung mit einem wässrigen Medium einer Umesterungsreaktion unterwirft, bei der - bezogen auf den zur α-Sulfonierung nicht verbrauchten SO₃-Anteil - wenigstens 0,5 Mol-Äquivalente Alkohol eingesetzt werden. Nach der DE-OS 35 38 910 können α-Sulfofettsäurealkylester-Salzpasten mit Feststoffgehalten oberhalb von 35 Gew.-% dadurch hergestellt werden, daß man die rohen Estersulfonate einer Umesterung im Sinne der DE-OS 34 32 324 unterwirft und dann bei der anschließenden Aufarbeitung durch Neutralisation mit oder ohne vorhergehender oder nachfolgender Bleiche in den wässrigen Pasten Feststoffgehalte von mehr als 35 Gew.-% einstellt.

Den Herstellungsverfahren für α-Sulfofettsäurealkylester-Alkalimetallsalzen, die bei der Aufarbeitung der rohen Sulfonierungsprodukte einen Zusatz von niederen Alkoholen vorsehen, haftet der Nachteil an, daß sie zu pastösen Zusammensetzungen führen, die mit den üblichen Bleichmitteln nicht mehr in hinreichendem Maß aufgehellt werden können.

Die vorliegende Erfindung geht auf die Aufgabenstellung zurück, ein Verfahren aufzufinden, das es gestattet, ohne Zusatz von Fremdstoffen wie längerkettigen aliphatischen Mono- und Polyalkoholen und deren Alkylenoxidanlagerungsprodukten, Alkylphenolen und deren Alkylenoxidanlagerungsprodukten oder kurzkettigen Alkoholen durch direkte Neutralisation der sauren α-Sulfofettsäurealkylester mit wässrigen Alkalimetallhydroxidlösungen zu fließ- und pumpfähigen Estersulfonatpasten mit WAS-Gehalten von 60 bis 70 Gew.-% zu gelangen. Die Lehre der Erfindung geht von der überraschenden Erkenntnis aus, daß dieses Ziel erreicht werden kann, wenn man bei der Neutralisation das saure Sulfonierungsprodukt und die wässrige Alkalimetallhydroxidlösung in eine bereits vorliegende wässrige Phase einträgt und dabei gleichzeitig dafür Sorge trägt, daß der pH-Wert der wässrigen Phase sich nur in einem ganz bestimmten Bereich bewegt.

Gegenstand der Erfindung ist ein Verfahren zur Herstellung hochkonzentrierter Pasten von α-Sulfofettsäurealkylester-Alkalimetallsalzen mit guter Bleichbarkeit durch Umsetzung von Fettsäurealkylestern mit gasförmigem SO₃, anschließende Nachreaktion in flüssiger Phase und Neutralisation mit wässrigen Alkalihydroxidlösungen, bei dem man bei der Neutralisation das Sulfierprodukt und wässrige Alkalimetallhydroxid-Lösung unter Aufrechterhaltung eines pH-Wertes im Bereich von 2 bis 8 in eine anfänglich 0 bis 55 Gew.-%, vorzugsweise 0 bis 54,9 Gew.-% Waschaktivsubstanz enthaltende wässrige Phase einleitet und Waschaktivsubstanzgehalte von 60 bis 70 Gew.-%, vorzugsweise 60 bis 65 Gew.-%, einstellt.

In einer speziellen Ausführungsform der Erfindung wird das Sulfierprodukt zusammen mit wässriger Alkalimetallhydroxidlösung in eine anfänglich 0 Gew.-% Waschaktivsubstanz enthaltende wässrige Phase, also in Wasser eingeleitet.

Werden zu Beginn der Neutralisation Waschaktivsubstanz enthaltende Lösungen als wässrige Phase eingesetzt, so ist der pH-Wert dieser Lösungen vorher auf einen Wert im Bereich von 2 bis 8 einzustellen.

In einer bevorzugten Ausführungsform der Erfindung wird während des Neutralisationsvorgangs in der wässrigen Phase bis zum Erreichen eines Gehaltes an Waschaktivsubstanz von 55 bis 65 Gew.-%, vorzugsweise von 48 bis 52 Gew.-%, ein pH-Wert im Bereich von 2 bis 6, vorzugsweise im Bereich von 3 bis 5 aufrechterhalten. Weiterhin ist es bevorzugt, nach dem Erreichen eines Gehaltes an Waschaktivsubstanz von 55 bis 65 Gew.-%, vorzugsweise von 48 bis 52 Gew.-%, in der wässrigen Phase einen pH-Wert von 5 bis 8, vorzugsweise im Bereich von 5,5 bis 7,5 einzustellen und aufrechtzuerhalten.

Die Neutralisation der α-Sulfofettsäurealkylester wird zweckmäßigerweise bei Temperaturen unterhalb von 95 °C, vorzugsweise bei Temperaturen im Bereich von 60 bis 90 °C durchgeführt. Als Neutralisationsbase ist Natriumhydroxid bevorzugt.

Der pH-Wert der wässrigen Phase wird vorzugsweise durch Variation der Volumenströme von α-Sulfofettsäurealkylester und wässriger Alkalimetallhydroxidlösung aufrecht erhalten.

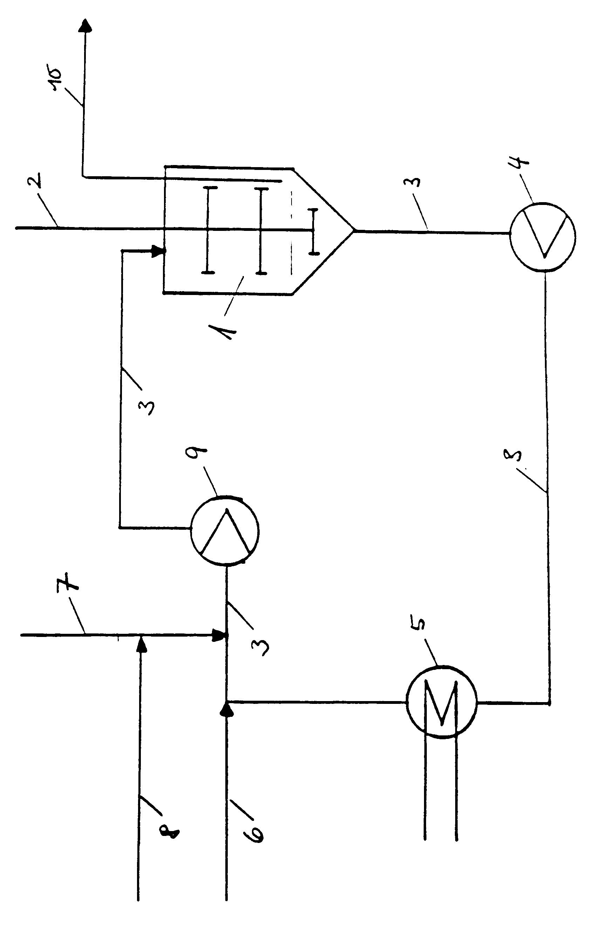

Die Neutralisation der sauren α-Sulfofettsäurealkylester wird zweckmäßigerweise in einer Neutralisationsschleife durchgeführt, deren schematischer Aufbau in der Fig. 1 dargestellt ist. Der überwiegende Teil der wässigen Phase befindet sich in dem Rührbehälter 1 und wird mit Hilfe des Rührers 2 ständig durchmischt. Über die Kreislaufleitung 3 wird daraus mittels der Umwälzpumpe 4 wässrige Phase laufend abgezogen und in dem für die Steuerung der Reaktionstemperatur vorgesehenen Kühler 5 im erforderlichen Ausmaß gekühlt. Der zu neutralisierende α-Sulfofettsäurealkylester wird über die Leitung 6 in den Strom der umgepumpten wässrigen Phase eingeleitet. Wässrige Alkalimetallhydroxidlösung einer Standard-Konzentration, beispielsweise 50 gew.-%ige Natriumhydroxidlösung, wird dem Kreislauf über die Leitung 7 zugeführt.

Durch Zufuhr von Wasser über die Leitung 8 kann die Konzentration der Standard-Alkalimetallhydroxidlösung vor deren Einspeisung in den Produktkreislauf auf den akut geforderten Wert vermindert werden. Das Gemisch aus saurem α-Sulfofettsäurealkylester, Alkalimetallhydroxidlösung und im Kreislauf geführter wässriger Phase gelangt dann zur weiteren Homogenisierung in den Mischer 9 und wird von dort über den letzten Abschnitt der Kreislaufleitung 3 in den Rührbehälter 1 gefördert. Die bei der Neutralisation entstehende α-Sulfofettsäurealkylester-Alkalimetallsalzpaste kann über die Leitung 10 abgezogen werden. Eine Neutralisationsschleife des hier beschriebenen Typs kann ausschließlich aus üblichen Vorrichtungen, Armaturen und Leitungen aufgebaut werden. Die erforderliche Überwachung von pH und Reaktionstemperatur und die Regulierung der Produkt- und Kühlmittelströme kann nach bekannten Verfahren der Meß- und Regeltechnik für chemische Verfahren erfolgen.

Unter Fettsäurealkylestern im Sinne der Erfindung werden Niedrigalkylester von gesättigten Fettsäuren verstanden, insbesondere Ester von Fettsäuren mit 10 bis 18 Kohlenstoffatomen und gesättigten aliphatischen Alkoholen mit 1 bis 4 Kohlenstoffatomen. Grundsätzlich kann man von einzelnen Fettsäurealkylestern ausgehen. In der Regel verwendet man als Ausgangsmaterial jedoch Estergemische, wie sie aus Fetten und Ölen natürlichen Ursprungs entweder durch Esterspaltung und nachfolgende Veresterung mit niederen Alkanolen oder durch Umesterung mit niederen Alkanolen nach bekannten Verfahren erhältlich sind, wobei die entsprechenden Fettsäuremetylestergemische wiederum bevorzugt sind. Soweit die auf diese Weise erhaltenen Fettsäureestergemische größere Anteile an Estern von Fettsäuren mit weniger als 10 Kohlenstoffatomen enthalten, werden diese "Vorlauffettsäureester" in der Regel durch Destillation abgetrennt. Die Fettsäureester dürfen außer der CH₂-Gruppe in α-Stellung zur Estergruppierung keine sulfatierbaren oder sulfonierbaren Gruppen enthalten. Aus diesem Grund kommen Hydroxyfettsäureester oder Hydroxyfettsäureester enthaltende Gemische nicht als Ausgangsmaterial in Betracht. Fettsäureestergemische, die nicht zu vernachlässigende Mengen an Estern ungesättigter Fettsäuren enthalten, insbesondere solche Ester, die eine Jodzahl über 5 aufweisen, sind erst nach einer Absättigung der Doppelbindungen im Zuge einer hydrierenden Härtung nach bekannten Verfahren als Ausgangsmaterial geeignet. Bei der hydrierenden Härtung werden die Jodzahlwerte der Estergemische vorzugsweise auf Werte von 0,2 und kleiner vermindert.

Die Sulfonierung der Fettsäureester erfolgt mit gasförmigem SO₃ als Sulfonierungsreagenz bei Temperaturen von 30 bis 100 °C. Dabei wird das SO₃ mit Luft oder Stickstoff verdünnt, vorzugsweise in Form eines Gasgemisches mit 1 bis 10 Vol.-% SO₃ mit den Fettsäureestern in Berührung gebracht. Die Menge des SO₃ wird so bemessen, daß das Sulfonierungsmittel in einem molaren Überschuß von mindestens 10 % - bezogen auf den Fettsäureester - vorliegt. Vorzugsweise liegt das Molverhältnis von Fettsäureester : SO₃ im Bereich von 1 : 1,2 bis 1 : 1,8 liegt. Diese Umsetzung kann in üblichen, für die Sulfonierung von organischen Verbindungen wie Fettalkoholen, Alkylbenzolen oder Olefinen geeigneten Reaktoren, insbesondere in Fallfilmreaktoren oder mehrstufigen Rührkesselkaskaden durchgeführt werden.

Das aus dem Sulfierreaktor kommende rohe Sulfonierungsprodukt weist noch nicht den gewünschten Sulfonierungsgrad auf. Aus diesem Grund wird das rohe Reaktionsprodukt unmittelbar nach der Sulfonierung einer geeigneten Vorrichtung zugeführt, in der es unter mechanischer Bewegung über einen Zeitraum von 20 bis 40 Minuten, vorzugsweise 25 bis 35 Minuten, einer temperaturkontrollierten Nachreaktion überlassen wird, bis der gewünschte Sulfiergrad erreicht ist. Die für diesen Reaktionsschritt notwendige Vorrichtung kann aus einem üblichen Reaktor mit Heiz- und Kühlkreislauf, einer üblichen temperierbaren Rohrschlange oder einer üblichen Rührkesselkaskade bestehen. Die Nachreaktion wird bei Temperaturen von 60 bis 100 °C durchgeführt. Die mechanische Bewegung des sulfonierten Produktes während der Nachreaktion kann durch Rühren, durch Zufuhr des Produktes unter Druck, durch den Einbau von Umlenkschikanen in die Vorrichtung oder, bei Verwendung einer Rohrschlange, durch Ausbildung einer turbulenten Strömung bewirkt werden. Die Nachreaktion des sulfonierten Produktes kann durch geeignete Wahl der genannten Parameter, insbesondere der Reaktionszeit, so gesteuert werden, daß ein Sulfiergrad von mindestens 90 %, vorzugsweise 94 bis 98 % erreicht wird.

Im Anschluß an die Nachreaktion wird das gealterte Sulfonierungsprodukt der Neutralisation gemäß der Erfindung zugeführt.

Die bei der Neutralisation erhaltenen Estersulfonatpasten stellen mehr oder weniger stark gefärbte Substanzen dar, die vor ihrer weiteren Verarbeitung gebleicht werden müssen. Die Bleichung erfolgt in an sich bekannter Weise unter Verwendung üblicher Bleichmittel wie wässrigen Lösungen von Natriumhypochlorit oder, vorzugsweise, wässrigen Lösungen von Wasserstoffperoxid. Die Bleichung mit Wasserstoffperoxid erfolgt bei pH-Werten im sauren Bereich. Vorzugsweise wird die Wasserstoffperoxidbleichung bei pH-Werten oberhalb von 5 durchgeführt.

### Beispiel 1

Als Ausgangsmaterial wurde ein technischer Palmitin-/Stearinsäuremethylester (in Gew.-% nach der Kettenlänge im Fettsäurerest: 0,2 C₁₂; 1,2 C₁₄; 61,4 C₁₆; 0,9 C₁₇; 35,9 C₁₈; 0,4 C₂₀; mittleres Molgewicht 281,5; Säurezahl 1,1; Jodzahl 0,1; Verseifungszahl 202,1) verwendet. Der Fettsäuremethylester wurde kontinuierlich in einem üblichen Fallfilmreaktor bei 80 °C mit einem SO₃-Luftgemisch (5 Vol.-% SO₃) im Molverhältnis 1 : 1,25 sulfoniert. Das resultierende Reaktionsgemisch wurde in einer aus vier Rührkesseln bestehenden Verweilzeitkaskade mit einer Verweilzeit von 25 Minuten einer Nachreaktion unterworfen. Danach betrug die Säurezahl des Sulfonierungsproduktes 198. Der Sulfonierungsgrad lag bei 96 %.

In einer Neutralisationsschleife des beschriebenen Typs wurden 195 kg Wasser eingebracht und umgepumpt. In den Kreislauf der wässrigen Phase wurden 1090 kg des vorstehend beschriebenen gealterten Sulfonierungsproduktes und 300 kg 50 gew.-%igen Natriumhydroxidlösung zunächst mit einer solchen Geschwindigkeit eingespeist, daß in der wässrigen Phase ein pH-Wert von 4,5 bis 5,5 aufrechterhalten wurde. Sobald der WAS-Gehalt der wässrigen Phase 63 Gew.-% erreicht hatte, wurden die Zuflußgeschwindigkeiten des Sulfonierungsproduktes und der Natriumhydroxidlösung so einreguliert, daß der pH-Wert in der wässrigen Phase bei 6 lag. Nachdem das gesamte saure Sulfonierungsprodukt dem Neutralisationskreislauf zugeführt war, wurde der pH-Wert der wässrigen Phase durch Zugabe der restlichen Menge Natriumhydroxidlösung auf 7,5 angehoben. Während der gesamten Neutralisation wurde die Reaktionstemperatur auf 80 bis 83 °C gehalten. Die wässrige Phase konnte während des gesamten Neutralisationsvorgangs problemlos gerührt und umgepumpt werden.

Es wurden 1585 kg rühr- und pumpfähige α-Sulfofettsäuremethylester-Natriumsalzpaste mit einem WAS-Gehalt von 63,1 Gew.-% (51,6 Gew.-% α-Sulfofettsäuremethylester-Natriumsalz und 11,5 Gew.-% α-Sulfofettsäuredinatriumsalz) erhalten.

### Beispiel 2

792 kg der α-Sulfofettsäuremethylester-Natriumsalzpaste aus Beispiel 1 wurden durch Eintragen in 792 kg Wasser zu einer Raste mit einem WAS-Gehalt von 31,6 Gewichtsprozent verdünnt. Die verdünnte Paste wurde als wässrige Phase in die Neutralisationsschleife eingebracht und umgepumpt. In den Kreislauf der wässrigen Phase wurden 792 kg des gealterten Sulfonierungsproduktes aus Beispiel 1 und 150 kg 50 gew.-%ige Natriumhydroxidlösung zunächst mit einer solchen Geschwindigkeit eingespeist, daß in der wässrigen Phase ein pH-Wert von 5 aufrechterhalten wurde. Sobald der WAS-Gehalt der wässrigen Phase 60 Gew.-% erreicht hatte, wurden die Mengenflüsse so einreguliert, daß der pH-Wert in der wässrigen Phase bei 6 lag. Nachdem das gesamte saure Sulfonierungsprodukt in die wässrige Phase eingetragen war, wurde der pH-Wert durch Zugabe der restlichen Menge Natriumhydroxid auf 7,5 gebracht. Die Reaktionstemperatur betrug 90 bis 93 °C. Die wässrige Phase konnte während des gesamten Neutralisationsvorgangs ohne Schwierigkeiten gerührt und umgepumpt werden.

Es wurden 1585 kg rühr- und pumpfähige α-Sulfofettsäuremethylester-Natriumsalzpaste mit einem Waschaktivsubstanzgehalt von 63,1 Gew.-% (51,6 Gew.-% α-Sulfofettsäuremethylester-Natriumsalz und 11,5 Gew.-% α-Sulfofettsäuredinatriumsalz) erhalten.

## Patentansprüche

1. Verfahren zur Herstellung hochkonzentrierter Pasten von α-Sulfofettsäurealkylester-Alkalimetallsalzen mit guter Bleichbarkeit durch Umsetzung von Fettsäurealkylestern mit gasförmigem SO₃, anschließende Nachreaktion in flüssiger Phase und Neutralisation mit wässrigen Alkalihydroxidlösungen, dadurch gekennzeichnet, daß man bei der Neutralisation das Sulfierprodukt und wässrige Alkalimetallhydroxid-Lösung unter Aufrechterhaltung eines pH-Wertes im Bereich von 2 bis 8 in eine anfänglich 0 bis 55 Gew.-% Waschaktivsubstanz enthaltende wässrige Phase einleitet und Waschaktivsubstanzgehalte von 60 bis 70 Gew.-%, vorzugsweise 60 bis 65 Gew.-%, einstellt.

2. Verfahren nach Anspruch 1, dadurch gekennzeichnet, daß man das Sulfierprodukt zusammen mit wässriger Alkalimetallhydroxidlösung in eine anfänglich 0 Gew.-% Waschaktivsubstanz enthaltende wässrige Phase einleitet.

3. Verfahren nach mindestens einem der Ansprüche 1 und 2, dadurch gekennzeichnet, daß man in der wässrigen Phase bis zum Erreichen eines Gehaltes an Waschaktivsubstanz von 55 bis 65 Gew.-%, vorzugsweise von 60 bis 65 Gew.-%, einen pH-Wert im Bereich von 2 bis 6, vorzugsweise im Bereich von 3 bis 5 aufrecht erhält.

4. Verfahren nach mindestens einem der Ansprüche 1 bis 3, dadurch gekennzeichnet, daß man in der wässrigen Phase nach dem Erreichen eines Gehaltes an Waschaktivsubstanz von 55 bis 65 Gew.-%, vorzugsweise von 60 bis 65 Gew.-%, einen pH-Wert im Bereich von 5 bis 8, vorzugsweise einen im Bereich von 5,5 bis 7,5, aufrecht erhält.

5. Verfahren nach mindestens einem der Ansprüche 1 bis 4, dadurch gekennzeichnet, daß man die Neutralisation bei Temperaturen unterhalb von 95 °C, vorzugsweise bei Temperaturen im Bereich von 60 bis 90 °C, durchführt.

6. Verfahren nach mindestens einem der Ansprüche 1 bis 5, dadurch gekennzeichnet, daß man den pH-Wert der wässrigen Phase durch Variation der Volumenströme von α-Sulfofettsäurealkylester und wässriger Alkalimetallhydroxidlösung steuert.

7. Verfahren nach mindestens einem der Ansprüche 1 bis 6, dadurch gekennzeichnet, daß man als Ausgangsmaterial Fettsäuremethylester einsetzt.

8. Verfahren nach mindestens einem der Ansprüche 1 bis 7, dadurch gekennzeichnet, daß man als Ausgangsmaterial durch Umesterung von natürlichen Fetten und/oder Ölen mit Methanol erhältliche Fettsäuremethylestergemische einsetzt.

9. Verfahren nach mindestens einem der Ansprüche 1 bis 8, dadurch gekennzeichnet, daß man die Fettsäurealkylester in einem üblichen Sulfierreaktor mit einem mindestens 10 %-igen molaren Überschuß an SO₃ zur Umsetzung bringt.

10. Verfahren nach mindestens einem der Ansprüche 1 bis 9, dadurch gekennzeichnet, daß man als Sulfierreagenz ein SO₃/Luft- oder SO₃/Stickstoffgemisch mit 1 bis 10 Vol.-% SO₃ einsetzt.

11. Verfahren nach mindestens einem der Ansprüche 1 bis 10, dadurch gekennzeichnet, daß man die Umsetzung mit SO₃ in einem Molverhältnis Fettsäureester : SO₃ im Bereich von 1 : 1,2 bis 1 : 1,8 durchführt.

12. Verfahren nach mindestens einem der Ansprüche 1 bis 11, dadurch gekennzeichnet, daß man die Umsetzung mit SO₃ in einem Fallfilmreaktor oder in einer mehrstufigen Sulfierkaskade durchführt.

13. Verfahren nach mindestens einem der Ansprüche 1 bis 12, dadurch gekennzeichnet, daß man das aus dem Sulfierreaktor kommende rohe Sulfierprodukt in einer geeigneten Vorrichtung unter mechanischer Bewegung einer temperaturkontrollierten Nachreaktion bis zum Erreichen des gewünschten Sulfiergrades überläßt.

14. Verfahren nach mindestens einem der Ansprüche 1 bis 13, dadurch gekennzeichnet, daß man die Nachreaktion in einem Reaktor mit Heiz- und Kühlkreislauf, einer temperierbaren Rohrschlange oder einer Rührkesselkaskade durchführt.

15. Verfahren nach mindestens einem der Ansprüche 1 bis 14, dadurch gekennzeichnet, daß man die Nachreaktion bei 60 bis 100 °C durchführt.

16. Verfahren nach mindestens einem der Ansprüche 1 bis 15, dadurch gekennzeichnet, daß man bei der Nachreaktion die mechanische Bewegung des Sulfierproduktes durch Rühren, Zufuhr des Produktes unter Druck, Einbau von Umlenkschikanen in die Vorrichtung oder Ausbildung einer turbulenten Strömung in der Rohrschlange bewirkt.

17. Verfahren nach mindestens einem der Ansprüche 1 bis 16, dadurch gekennzeichnet, daß man das Sulfierprodukt so lange nachreagieren läßt, bis der Sulfiergrad mindestens 90 %, vorzugsweise 94 bis 98 % beträgt.

18. Verfahren nach mindestens einem der Ansprüche 1 bis 17, dadurch gekennzeichnet, daß man die erhaltenen α-Sulfofettsäurealkylester-Alkalimetallsalz-Pasten durch Bleichen weiterbehandelt.

19. Verfahren nach mindestens einem der Ansprüche 1 bis 18, dadurch gekennzeichnet, daß man die Pasten einer Bleiche unter Verwendung wässriger Lösungen von Wasserstoffperoxid unterwirft.

20. Verfahren nach mindestens einem der Ansprüche 1 bis 19, dadurch gekennzeichnet, daß man die Bleichung bei einem pH-Wert oberhalb von 5,0 durchführt.

## Claims

1. A process for the production of highly concentrated, readily bleachable pastes of α-sulfofatty acid alkyl ester alkali metal salts by reaction of fatty acid alkyl esters wit]i gaseous SO₃, subsequent after-reaction in liquid phase and neutralization with aqueous alkali hydroxide solutions, characterized in that the sulfonation product and aqueous alkali metal hydroxide solution are introduced during neutralization into an aqueous phase initially containing 0 to 55% by weight washing-active substance at a pH value in the range from 2 to 8 and washing-active substance contents of 60 to 70% by weight and preferably 60 to 65% by weight are established.

2. A process as claimed in claim 1, characterized in that the sulfonation product and the aqueous alkali metal hydroxide solution are introduced into an aqueous phase initially containing 0% by weight washing-active substance.

3. A process as claimed in at least one of claims 1 and 2, characterized in that a pH value in the range from 2 to 6 and preferably in the range from 3 to 5 is maintained in the aqueous phase until a content of washing-active substance of 55 to 65% by weight and preferably 60 to 65% by weight is reached.

4. A process as claimed in at least one of claims 1 to 3, characterized in that a pH value in the range from 5 to 8 and preferably in the range from 5.5 to 7.5 is maintained in the aqueous phase after a washing-active substance content of 55 to 65% by weight and preferably 60 to 65% by weight has been reached.

5. A process as claimed in at least one of claims 1 to 4, characterized in that neutralization is carried out at temperatures below 95°C and preferably at temperatures in the range from 60 to 90°C.

6. A process as claimed in at least one of claims 1 to 5, characterized in that the pH value of the aqueous phase is controlled by variation of the feed rates of α-sulfofatty acid alkyl ester and aqueous alkali metal hydroxide solution.

7. A process as claimed in at least one of claims 1 to 6, characterized in that fatty acid methyl ester is used as the starting material.

8. A process as claimed in at least one of claims 1 to 7, characterized in that fatty acid methyl ester mixtures obtainable by transesterification of natural fats and/or oils with methanol are used as the starting material.

9. A process as claimed in at least one of claims 1 to 8, characterized in that the fatty acid alkyl esters are reacted with an at least 10% molar excess of SO₃ in a standard sulfonation reactor.

10. A process as claimed in at least one of claims 1 to 9, characterized in that an SO₃/air mixture or an SO₃/nitrogen mixture containing 1 to 10% by volume SO₃ is used as the sulfonation reagent.

11. A process as claimed in at least one of claims 1 to 10, characterized in that the reaction with SO₃ is carried out in a molar ratio of fatty acid ester to SO₃ of 1 : 1.2 to 1 : 1.8.

12. A process as claimed in at least one of claims 1 to 11, characterized in that the reaction with SO₃ is carried out in a falling film reactor or in a multiple-stage sulfonation cascade.

13. A process as claimed in at least one of claims 1 to 12, characterized in that the crude sulfonation product coming from the sulfonation reactor is subjected to a temperature-controlled after-reaction in a suitable apparatus, in which it is mechanically agitated, until the desired degree of sulfonation is reached.

14. A process as claimed in at least one of claims 1 to 13, characterized in that the after-reaction is carried out in a reactor comprising a heating and cooling circuit, a temperature-controlled pipe coil or a cascade of stirred tanks.

15. A process as claimed in at least one of claims 1 to 14, characterized in that the after-reaction is carried out at 60 to 100°C.

16. A process as claimed in at least one of claims 1 to 15, characterized in that the sulfonation product is mechanically agitated during the after-reaction by stirring, by introduction of the product under pressure, by installation of the chicane-like baffles in the apparatus or by generation of a turbulent flow in the pipe coil.

17. A process as claimed in at least one of claims 1 to 16, characterized in that the sulfonation product is left to after-react until the degree of sulfonation is at least 90% and preferably 94 to 98%.

18. A process as claimed in at least one of claims 1 to 17, characterized in that the α-sulfofatty acid alkyl ester alkali metal salt pastes obtained are further treated by bleaching.

19. A process as claimed in at least one of claims 1 to 18, characterized in that the pastes are bleached using agueous solutions of hydrogen peroxide.

20. A process as claimed in at least one of claims 1 to 19, characterized in that bleaching is carried out at a pH above 5.0.

## Revendications

1. Procédé de production de pâtes très concentrées d'esters alkyliques d'α-sulfoacides gras-sels de métal alcalin ayant une bonne aptitude au blanchiment par réaction d'esters alkyliques d'acides gras avec du SO₃ gazeux, puis post-réaction en phase liquide et neutralisation par des solutions aqueuses d'hydroxyde alcalin, caractérisé en ce que lors de la neutralisation on introduit le produit de sulfonation et de la solution aqueuse d'hydroxyde de métal alcalin en maintenant le pH entre 2 et 8 dans une phase aqueuse contenant au départ de 0 à 55 % en poids de substance active de lavage et on ajuste les teneurs en substance active de lavage entre 60 et 70 % en poids, de préférence 60 à 65 % en poids.

2. Procédé selon la revendication 1, caractérisé en ce qu'on introduit le produit de sulfonation avec la solution aqueuse d'hydroxyde de métal alcalin dans une phase aqueuse contenant au début 0 % en poids de substance active de lavage.

3. Procédé selon au moins l'une des revendications 1 et 2, caractérisé en ce qu'on maintient dans la phase aqueuse le pH dans l'intervalle de 2 à 6, de préférence dans l'intervalle de 3 à 5 jusqu'à l'obtention d'une teneur en substance active de lavage de 55 à 65 % en poids, de préférence 60 à 65 % en poids.

4. Procédé selon au moins l'une des revendications 1 à 3, caractérisé en ce qu'après l'obtention dans la phase aqueuse d'une teneur en substance active de lavage de 55 à 65 % en poids, de préférence de 60 à 65 % en poids, on obtienne un pH dans l'intervalle de 5 à 8, de préférence dans l'intervalle de 5,5 à 7,5.

5. Procédé selon au moins l'une des revendications 1 à 4 caractérisé en ce qu'on réalise la neutralisation à des températures inférieures à 95°C, de préférence à des températures dans l'intervalle de 60 à 90°C.

6. Procédé selon au moins l'une des revendications 1 à 5, caractérisé en ce qu'on régule le pH de la phase aqueuse par variation des débits volumiques des esters alkyliques d'α-sulfoacides gras et de solution aqueuse d'hydroxyde de métal alcalin.

7. Procédé selon au moins l'une des revendications 1 à 6, caractérisé en ce qu'on utilise comme matériau de départ de l'ester méthylique d'acide gras.

8. Procédé selon au moins l'une des revendications 1 à 7, caractérisé en ce qu'on utilise comme matériau de départ des mélanges d'esters méthyliques d'acides gras qu'on obtient par transestérification de graisses et/ou d'huiles naturelles avec du méthanol.

9. Procédé selon au moins l'une des revendications 1 à 8 caractérisé en ce qu'on fait réagir les esters alkyliques d'acides gras dans un réacteur usuel de sulfonation avec un excès d'au moins 10 % molaire de SO₃.

10. Procédé selon au moins l'une des revendications 1 à 9, caractérisé en ce qu'on utilise comme réactif de sulfonation un mélange SO₃/air ou SO₃/azote avec une teneur de 1 à 10 % en volume de SO₃.

11. Procédé selon au moins l'une des revendications 1 à 10 caractérisé en ce qu'on réalise la réaction avec SO₃ en une proportion molaire esters d'acides gras : SO₃ dans l'intervalle de 1 : 1,2 à 1 : 1,8.

12. Procédé selon au moins l'une des revendications 1 à 11, caractérisé en ce qu'on réalise la réaction avec SO₃ dans un réacteur à ruissellement ou dans une cascade de sulfonation à plusieurs étages.

13. Procédé selon au moins l'une des revendications 1 à 12, caractérisé en ce qu'on place sans mouvement mécanique dans un dispositif approprié le produit brut de sulfonation provenant du réacteur de sulfonation pour une post-réaction à température contrôlée jusqu'à obtention du taux de sulfonation souhaité.

14. Procédé selon au moins l'une des revendications 1 à 13, caractérisé en ce qu'on réalise la postréaction dans un réacteur avec un circuit de chauffage et de refroidissement, un serpentin d'équilibrage de température ou une cascade de réacteurs agités.

15. Procédé selon au moins l'une des revendications 1 à 14, caractérisé en ce qu'on réalise la post-réaction entre 60 et 100°C.

16. Procédé selon au moins l'une des revendications 1 à 15, caractérisé en ce que lors de la postréaction on obtient le mouvement mécanique du produit de sulfonation par agitation, introduction du produit sous pression, implantation des chicanes de déviation dans le dispositif ou création d'un écoulement turbulent dans le serpentin.

17. Procédé selon au moins l'une des revendications 1 à 16, caractérisé en ce qu'on fait post-réagir le produit de sulfonation jusqu'à ce que le taux de sulfonation atteigne au moins 90 %, de préférence 94 à 98 %.

18. Procédé selon au moins l'une des revendications 1 à 17, caractérisé en ce qu'on traite encore les pâtes obtenues d'esters alkyliques d'α-sulfoacides gras-sels de métal alcalin par blanchiment.

19. Procédé selon au moins l'une des revendications 1 à 18, caractérisé en ce qu'on soumet les pâtes à un blanchiment en utilisant des solutions aqueuses de peroxyde d'hydrogène .

20. Procédé selon au moins l'une des revendications 1 à 19, caractérisé en ce qu'on réalise le blanchiment à un pH supérieur à 5,0.
